(19) Europäisches Patentamt
European Patent Office
Office européen des brevets

(11) Veröffentlichungsnummer : **0 116 323**
**B1**

(12) # EUROPÄISCHE PATENTSCHRIFT

(45) Veröffentlichungstag der Patentschrift : **20.08.86**

(51) Int. Cl.[4] : **C 07 C 47/52**, C 07 C 45/00, C 07 C149/36

(21) Anmeldenummer : **84100795.8**

(22) Anmeldetag : **26.01.84**

(54) **Verfahren zur Herstellung von aromatischen Aldehyden.**

(30) Priorität : **08.02.83 DE 3304202**

(43) Veröffentlichungstag der Anmeldung : **22.08.84 Patentblatt 84/34**

(45) Bekanntmachung des Hinweises auf die Patenterteilung : **20.08.86 Patentblatt 86/34**

(84) Benannte Vertragsstaaten : **CH DE FR GB LI**

(56) Entgegenhaltungen :
**EP-A- 0 035 624**
**DE-A- 2 732 227**
**US-A- 3 833 660**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Kysela, Ernst, Dr.**
**Virchowstrasse 14**
**D-5060 Bergisch-Gladbach 3 (DE)**
Erfinder : **Klauke, Erich, Dr.**
**Eichendorffweg 8**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 116 323 B1**

Jouve, 18, rue St-Denis, 75001 Paris, France

**Beschreibung**

Die Erfindung betrifft ein Verfahren zur Herstellung von teilweise neuen aromatischen Aldehyden durch Formylierung der entsprechenden aromatischen Verbindungen mit Urotropin in Gegenwart von Fluorwasserstoff.

Es ist bekannt, Phenole oder Alkylaniline in Gegenwart von Glyzerin-Borsäure oder Essigsäure mit Urotropin zu formylieren (Houben-Weyl VII/1, 199 (1954), Tetrahedron 24, 5001-5010 (1968)). Alkylphenole und alkylbenzole können in Gegenwart von Trifluoressigsäure mit Urotropin formyliert werden (J.O.C. 37 (24) 3973 (1972)). In Gegenwart von Polyphosphorsäure können Chlor- oder Nitro-Phenole sowie Halogenbenzole mit Urotropin formyliert werden (J.C.S. [London], 10741 (1963)).

Die bekannten Methoden sind im allgemeinen nur zur Formylierung elektronenreicher Aromaten geeignet.

Die Ausbeuten an Halogenbenzaldehyden sind unbefriedigend.

Es wurde ein Verfahren zur Formylierung von aromatischen Verbindungen der Formel

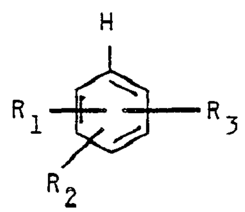

in der

$R_1$ Halogen oder durch Fluor substituiertes Niederalkyl, Niederalkoxy odeer Niederalkylthio und

$R_2$ und $R_3$ gleich oder verschieden sind und Wasserstoff, Halogen, Hydroxy oder durch Fluor substituiertes Niederalkyl, Niederalkoxy oder Niederalkylthio bedeuten

mit Urotropin gefunden, das dadurch gekennzeichnet ist, daß man die Formylierung in Gegenwart von fluorwasserstoff, gegebenenfalls bei erhöhter Temperatur und erhöhtem Druck, durchführt.

Das erfindungsgemäße Verfahren ist zur Formylierung elektronenärmerer aromatischer Verbindungen geeignet.

Halogen bedeutet hierbei erfindungsgemäß Fluor, Chlor, Brom und Jod, bevorzugt Fluor, Chlor und Brom.

Niederalkyl sowie der Alkylteil bei Niederalkoxy und bei Niederalkylthio kann hierbei ein geradkettiger oder verzweigter Kohlenwasserstoffrest mit 1 bis etwa 8 Kohlenstoffatomen, bevorzugt 1 bis 4 Kohlenstoffatomen, sein.

Beispielsweise seien die folgenden Niederalkylreste genannt. Methyl, Ethyl, Propyl, Isopropyl, Butyl, Isobutyl, Pentyl, Isopentyl, Hexyl, Isohexyl, Heptyl, Isoheptyl, Octyl und Isooctyl.

Beispielsweise seien die folgenden Niederalkoxyreste genannt : Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy und Isooctoxy.

Beispielsweise seien die folgenden Niederalkylthioreste genannt : Methylthio, Ethylthio, Propylthio, Isopropylthio, Butylthio, Isobutylthio, Pentylthio, Isopenthylthio, Hexylthio, Isohexylthio, Heptylthio, Isoheptylthio, Octylthio und Isooctylthio.

Die genannten Reste sind teilweise oder vollständig durch Fluor substituiert. Bei teilweiser Substition können die Reste bis zu n-1 Fluoratome enthalten (wenn n die Zahl der Wasserstoffatome im entsprechenden Alkylrest bedeutet) und alle statistisch denkbaren Positionen einnehmen.

Beispielsweise seien die folgenden fluorierten Niederalkylverbindungen genannt : Trifluormethyl, Perfluorethyl, Perfluorisopropyl, 1,1,2,2-Tetrafluorethyl, Hexafluorisopropyl, 1,1,1-Trifluorpropyl.

Beispielsweise seien die folgenden fluorierten Niederalkoxyverbindungen genannt : Trifluormethoxy, Perfluorethoxy, Perfluorisopropoxy, 1,1,2,2,-Tetrafluorethoxy, Hexafluorisopropoxy, 3,3,3-Trifluor-n-propoxy, 1,1,1-Trifluor-5-methyl-methoxy.

Beispielsweise seien die folgenden Fluoralkylthioverbindungen genannt : Trifluormethylthio, Perfluorethylthio, Perfluorisopropylthio, 1,1,2,2,-Tetrafluorethylthio, Hexafluorisopropoxy, 3,3,3-Trifluor-n-propylthio, 1,1,1-Trifluor-5-methylhexylthio.

Insbesondere bevorzugt für das erfindungsgemäße Verfahren werden aromatische Verbindungen, die durch Fluor enthaltende Reste substituiert sind.

Die aromatischen Verbindungen für das erfindungsgemäße Verfahren sind an sich bekannt.

Als Flußsäure für das erfindungsgemäße Verfahren wird im allgemeinen wasserfreie Flußsäure eingesetzt.

Für das erfindungsgemäße Verfahren setzt man im allgemeinen 0,5 bis 5 Mol, bevorzugt 1 bis 2 Mol, Urotropin bezogen auf 1 Mol der aromatischen Verbindung ein.

Für das erfindungsgemäße Verfahren setzt man im allgemeinen 10 bis 100 Mol, bvorzugt 25 bis 50 Mol, Flußsäure bezogen auf 1 Mol der aromatischen Verbindung ein.

Das erfindungsgemäße Verfahren wird im allgemeinen im Druckbereich von Normaldruck bis 25 bar, bevorzugt von 2 bis 15 bar, durchgeführt. Die erfindungsgemäßen Drucke stellen sich beispielsweise

durch den Eigendruck ein, der entsteht, wenn man im erfindungsgemäßen Temperaturbereich das Verfahren in einem Autoklaven durchführt.

Das erfindungsgemäße Verfahren kann beispielsweise wie folgt durchgeführt werden :

In einem Autoklaven legt man die aromatische Verbindung, das Urotropin und die Flußsäure vor. Das Reaktionsgemisch wird auf die Reaktionstemperatur erhitzt, wobei sich der entsprechende Reaktionsdruck einstellt.

Nach Beendigung der Umsetzung wird der Restdruck entspannt und das Reaktionsgemisch wäßrig aufgearbeitet.

Nach dem erfindungsgemäßen Verfahren können aromatische Aldehyde der Formel

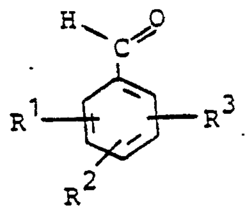

(III)

in der $R^1$ bis $R^3$ die obengenannte Bedeutung haben, hergestellt werden.

Nach dem erfindungsgemäßen Verfahren können neue aromatische Aldehyde der Formel

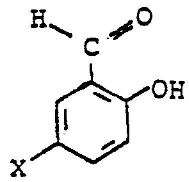

(IV)

in der X Trifluormethylthio oder Trifluormethoxy bedeutet, hergestellt werden.

Die nach dem erfindungsgemäßen Verfahren hergestellten aromatischen Aldehyde, im besonderen die neuen aromatischen Aldehyde der Formel (IV) sind Zwischenprodukte für die Herstellung von Pflanzenschutzmitteln, insbesondere Insektizide. So kann man beispielsweise durch Umsetzung mit einem Hydroxylamin in einer ersten Stufe ein Oxim herstellen und dann in einer zweiten Stufe über eine Etherbindung einen Phosphonrest einführen.

## Beispiel 1

4-Trifluormethoxybenzaldehyd (A) (2-Trifluormethoxybenzaldehyd (B)

162 g (1 Mol) Trifluormethoxybenzol und 140 g (1 Mol) Urotropin werden in einem $V_4A$ Rührautoklaven unter Solekühlung (— 5 °C) mit 0,5 l (25 Mol) HF versetzt. Die Apparatur wird druckdicht verschlossen und für 5 Stunden auf 80 °C erwärmt. Es stellt sich ein Druck von 6 bis 7 bar ein. Nach Reaktionsende das rohe, auf 25 °C abgekühlte Reaktionsgemisch in 1 l Wasser einrühren, 15 Minuten bei 25 °C rühren, dann organische Anteile isolieren, die wäßrige Phase mit Methylenchlorid extrahieren. Aus den gewaschenen, organischen Phasen können durch Rohdestillation 135 g Produkt gewonnen werden ($Kp_{18mbar}$ 72-74 °C). Es besteht zu 93 % aus 4-Trifluormethoxy-Benzaldehyd ($Kp_{20mbar}$ 78-80 °C) und 6 % aus 2-Trifluormethoxy-Benzaldehyd ($Kp_{20mbar}$ 60-61 °C). Beide können durch fraktionierte Destillation getrennt werden.

## Beispiel 2

4-Trifluormethoxybenzaldehyd (2-Trifluormethoxybenzaldehyd)

Analog Beispiel 1 wurden 162 g (1 Mol) Trifluormethoxybenzol bei 100 °C umgesetzt. Die Rohdestillation lieferte 119 g Produkt ($Kp_{18mbar}$ 72-74 °C), bestehend aus 90 % 4-Trifluormethoxybenzaldehyd und 8 % 2-Trifluormethoxybenzaldehyd.

## Beispiel 3

Analog Beispiel 1 wurden 162 g (1 Mol) Trifluormethoxybenzol bei 60 °C und in Gegenwart von 1 l (50 Mol) HF eingesetzt. Die Rohdestillation lieferte 115 g Produkt ($Kp_{18}$mbar 72-74 °C).

## Beispiel 4

Analog Beispiel 1 wurden 162 g (1 Mol) Trifluormethoxybenzol bei 100 °C und in Gegenwart von 0,5 Mol Urotropin umgesetzt. Die Rohdestillation lieferte 82 g Produkt ($Kp_{18mbar}$ 72-74 °C).

Beispiel 5

2-Methyl-5-fluor-benzaldehyd (2-Fluor-5-methyl-benzaldehyd)

Analog Beispiel 1 wurden 110 g (1 Mol) 4-Fluortoluol umgesetzt. Die Rohdestillation lieferte 80 g Produkt ($Kp_{20mbar}$ 83-85 °C) bestehend aus 80 % 2-Methyl-5-fluor, benzaldehyd ($Kp_{20mbar}$ 82 °C) und 20 % 2-Fluor-5-methyl-benzaldehyd.

Beispiel 6

4-Fluorbenzaldehyd (2-Fluorbenzaldehyd)

Analog Beispiel 1 wurden 96 g Fluorbenzol (1 Mol) bei 100 °C umgesetzt. Die Rohdestillation lieferte 37 g Produkt ($Kp_{14mbar}$ 65-68 °C) bestehend aus 87 % 4-Fluorbenzaldehyd ($Kp_{14mbar}$ 64-65 °C) und 12 % 2-Fluorbenzaldehyd ($Kp_{14mbar}$ 59-60 °C), die destillativ getrennt werden können.

Beispiel 7

4-Methyl-5-trifluormethylbenzaldehyd

Analog Beispiel 1 wurden 160 g 2-Methylbenzotrifluorid (1 Mol) bei 100 °C umgesetzt. Die Destillation lieferte 50 g 4-Methyl-5-trifluormethylbenzaldehyd ($Kp_{20mbar}$ 86-87 °C).

Beispiel 8

2-Hydroxy-5-trifluormethoxybenzaldehyd

Analog Beispiel 1 wurden 178 g (1 Mol) 4-Trifluormethoxyphenol bei 100 °C umgesetzt. Die Destillation lieferte 84 g 2-Hydroxy-5-trifluormethoxybenzaldehyd ($Kp_{20mbar}$ 83-84 °C).

Beispiel 9

4-Trifluormethylthio-benzaldehyd

Analog Beispiel 1 wurden 178 g (1 Mol) Trifluormethylthiobenzol umgesetzt. Die Destillation lieferte 57 g 4-Trifluormethylthiobenzaldehyd ($Kp_{18mbar}$ 88-89 °C).

Beispiel 10

2-Hydroxy-5-trifluormethylthiobenzaldehyd

Analog Beispiel 1 wurden 194 g (1 Mol) 4-Trifluormethylthiophenol umgesetzt. Die Destillation lieferte 44 g 2-Hydroxy-5-trifluormethylthio-benzaldehyd ($Kp_{20mbar}$ 105-107 °C ; Fp 50-52 °C).

Beispiel 11

4-Chlorbenzaldehyd (2-Chlorbenzaldehyd)

Analog Beispiel 1 wurden 112 g (1 Mol) Chlorbenzol bei 80 °C umgesetzt. Die Rohdestillation lieferte 106 g Produkt ($Kp_{20mbar}$ 90-92 °C) bestehend aus 75 % 4-Chlorbenzaldehyd und 25 °C Chlorbenzaldehyd.

**Patentansprüche**

1. Verfahren zur Formylierung von aromatischen Verbindungen der Formel

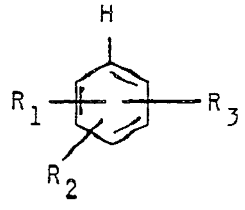

in der

$R_1$ Halogen oder durch Fluor substituiertes Niederalkyl, Niederalkoxy oder Nideralkylthio und

$R_2$ und $R_3$ gleich oder verschieden sind und Wasserstoff, Halogen, Hydroxy oder durch Fluor substituiertes Niederalkyl, Niederalkoxy oder Niederalkylthio bedeuten mit Urotropin, dadurch gekennzeichnet, daß man die Formylierung in Gegenwart von Fluorwasserstoff, gegebenenfalls bei erhöhter Temperatur und erhöhtem Druck, durchführt.

2. Verfahren nach Anspruch 1, dadurch gekennzeichnet, daß man die Formylierung im Druckbereich von Normaldruck bis 25 bar durchführt.

3. Verfahren nach den Ansprüchen 1 und 2, dadurch gekennzeichnet, daß man die Formylierung in dem Temperaturbereich von 0 bis 180 °C durchführt.

4. Verfahren nach den Ansprüchen 1 bis 3, dadurch gekennzeichnet, daß man 10 bis 100 Mol fluorwasserstoff bezogen auf 1 Mol der aromatischen Verbindung einsetzt.

5. Neue aromatische Aldehyde der Formel

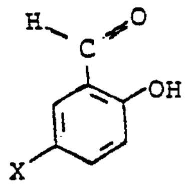

in der X Trifluormethylthio oder Trifluormethoxy bedeutet.

**Claims**

1. Process for formylating aromatic compounds of the formula

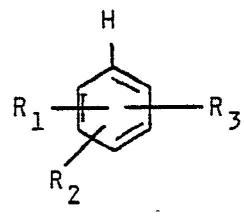

in which

$R_1$ is halogen or fluorine-substituted lower alkyl, lower alkoxy or lower alkylthio and

$R_2$ and $R_3$ are identical or different and denote hydrogen, halogen, hydroxyl or fluorine-substituted lower alkyl, lower alkoxy or lower alkylthio,

with urotropine, characterised in that the formylation is carried out in the presence of hydrogen fluoride, if appropriate at an elevated temperature and under an elevated pressure.

2. Process according to Claim 1, characterised in that the formylation is carried out in the pressure range from normal pressure to 25 bar.

3. Process according to Claims 1 and 2, characterised in that the formylation is carried out in the temperature range from 0 to 180 °C.

4. Process according to Claims 1 to 3, characterised in that 10 to 100 mol of hydrogen fluoride are used relative to 1 mol of the aromatic compound.

5. New aromatic aldehydes of the formula

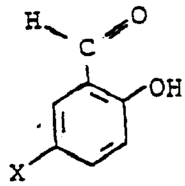

in which X denotes trifluoromethylthio or trifluoromethoxy.

**Revendications**

1. Procédé de formylation de composés aromatiques de formule

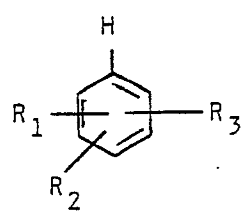

dans laquelle

$R_1$ désigne un halogène ou un groupe alkyle inférieur, alkoxy inférieur ou alkylthio inférieur substitué par du fluor et

$R_2$ et $R_3$ sont identiques ou différents et représentent l'hydrogène, un halogène, un groupe hydroxy ou un groupe alkyle inférieur, alkoxy inférieur ou alkylthio inférieur substitué par du fluor avec l'urotropine, caractérisé en ce qu'on conduit la formylation en présence de fluorure d'hydrogène, éventuellement à température élevée et sous pression élevée.

2. Procédé suivant la revendication 1, caractérisé en ce qu'on conduit la formylation dans l'intervalle de pression allant de la pression normale à 25 bars.

3. Procédé suivant les revendications 1 et 2, caractérisé en ce qu'on conduit la formylation dans l'intervalle de température de 0 à 180 °C.

4. Procédé suivant les revendications 1 à 3, caractérisé en ce qu'on utilise 10 à 100 moles de fluorure d'hydrogène par mole de composé aromatique.

5. Aldéhydes aromatiques nouveaux de formule

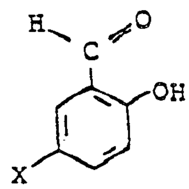

dans laquelle X désigne un groupe trifluorométhylthio ou trifluorométhoxy.